# EUROPEAN PATENT SPECIFICATION

(11) **EP 3 004 136 B1**
(45) Date of publication and mention of the grant of the patent: **12.04.2017**
(21) Application number: 14733870.1
(22) Date of filing: 29.05.2014
(51) Int. Cl.: C07K 5/103, C07K 16/06, C07K 7/06

(54) **PEPTOID AFFINITY LIGANDS FOR THE PURIFICATION OF ANTIBODIES OR ANTIBODY FRAGMENTS**
PEPTOIDE-AFFINITÄTSLIGANDE ZUR REINIGUNG VON ANTIKÖRPERN ODER DEREN FRAGMENTEN
LIGANDS D'AFFINITÉ Ä BASE PEPTOÏDIQUE POUR LA PURIFICATION D'ANTICORPS OU DE LEUR FRAGMENTS

(30) Priority: 31.05.2013 US 201361829712 P
(43) Date of publication of application: 13.04.2016
(73) Proprietor: Menegatti, Stefano, Goleta, California 93117 (US)
(72) Inventor: Menegatti, Stefano, Goleta, California 93117 (US)
(74) Representative: HGF Limited
(86) International application number: PCT/US2014/039995
(87) International publication number: WO 2014/194073

(56) References cited:
- N HEINE ET AL.: "Synthesis and screening of peptoid arrays on cellulose membranes", TETRAHEDRON, vol. 59, no. 50, 8 December 2003 (2003-12-08), pages 9919-9930, XP4474433, ELSEVIER SCIENCE PUBLISHERS ISSN: 0040-4020
- J M HAIGH ET AL.: "Affinity ligands for immunoglobulins based on the multicomponent Ugi reaction", JOURNAL OF CHROMATOGRAPHY B, vol. 877, no. 14-15, 15 May 2009 (2009-05-15), pages 1440-1452, XP26084905, ELSEVIER SCIENCE PUBLISHERS B.V. ISSN: 0021-9673
- S MENEGATTI ET AL.: "Purification iof polyclonal antibodies from Cohn fraction II + III, skim milk, and whey by affinity chromatography using a hexamer peptide ligand", JOURNAL OF SEPARATION SCIENCE, vol. 35, no. 22, November 2012 (2012-11), pages 3139-3148, XP002729498, WILEY VCH, WEINHEIM. ISSN: 0935-6304

## Description

### Background of the Invention

Monoclonal antibodies and Fc-fusion proteins have emerged as an important class of therapeutic proteins for the treatment of a number of unmet diseases such as cancer, autoimmune diseases, immunodeficiency, skin disorders and neurological disorders. These products account for 40% of the overall pharmaceutical market with a volume of $ 35 billion in 2011. However, therapies based on antibodies are very expensive to consumers. Their high price is due in part to the high cost of isolation and purification of these biomolecules. A major contribution to the purification costs results from the ubiquitous use of Protein A or Protein G affinity chromatography capture step. Despite their high selectivity for IgG, these protein ligands suffer from high cost and low chemical and biochemical stability. The average cost of Protein A / G - based chromatographic media ranges between $ 8,000 - 15,000 per liter of resin. Protein ligands show poor chemical resistance towards the alkaline (0.1 - 1.0 M NaOH) cleaning procedures periodically applied for the removal of contaminants. Further they are prone to proteolytic degradation by enzymes present in the feed. Both these chemical and enzymatic agents can cause ligand degradation and leakage from the resin, resulting in shorter column lifetime and the potential presence of toxic and immunogenic leachates in the product mainstream.

### Summary of the Invention

Extensive research has been carried out in both industry and academia to discover inexpensive yet selective and robust affinity ligands for antibodies. A variety of synthetic compounds, including triazinic scaffolds, amino acids and peptides have been proposed for the selection of affinity ligands for downstream processing of biotherapeutics. A class that has not been yet explored for the design of affinity ligands is represented by peptoids, even though this class of compounds possesses ideal characteristics for such application. First, the display of functional groups on peptoids resembles that of peptides, implying that peptoids can possess levels of affinity and selectivity comparable to those shown by peptide ligands. Further, owing to the so-called "sub-monomer" protocol of synthesis, which employs amines, peptoids can explore a much wider chemical diversity than that available to the above mentioned compounds. This enables fine tuning their composition to achieve higher target specificity and avidity. Finally, peptoids are completely resistant to proteolysis and are therefore advantageous for the purification of antibodies from fluids containing active enzymes, like whole plasma and its fractions.

Peptoids are therefore an economical alternative to Protein A. Besides the purification of biopharmaceuticals, these ligands can find further applications in areas such as diagnostics and process control. To the best of our knowledge, peptoid ligands have not been suggested for the purification of IgG.

Accordingly, a first aspect of the invention is a peptoid ligand that specifically binds to an antibody such as IgG, and/or an antibody Fc fragment, and/or an Fc-fusion protein. Such peptoid ligands are in some embodiments from 4 to 7 residues or monomers in length. Such peptoid ligands are optionally, but in some embodiments preferably, coupled to a solid support.

A further aspect of the invention is a method of binding an antibody or antibody Fc fragment or an Fc-fusion protein from a liquid composition containing the same, comprising the steps of: (a) providing a solid support comprising a peptoid ligand bound thereto as described herein, (b) contacting said composition to said solid support so that antibody or Fc fragments bind to said compound; and (c) separating said liquid composition from said solid support, with said antibody or Fc fragment bound to said solid support.

The foregoing and other objects and aspects of the present invention are explained in detail in the specification set forth below.

### Brier Description of the Drawlings

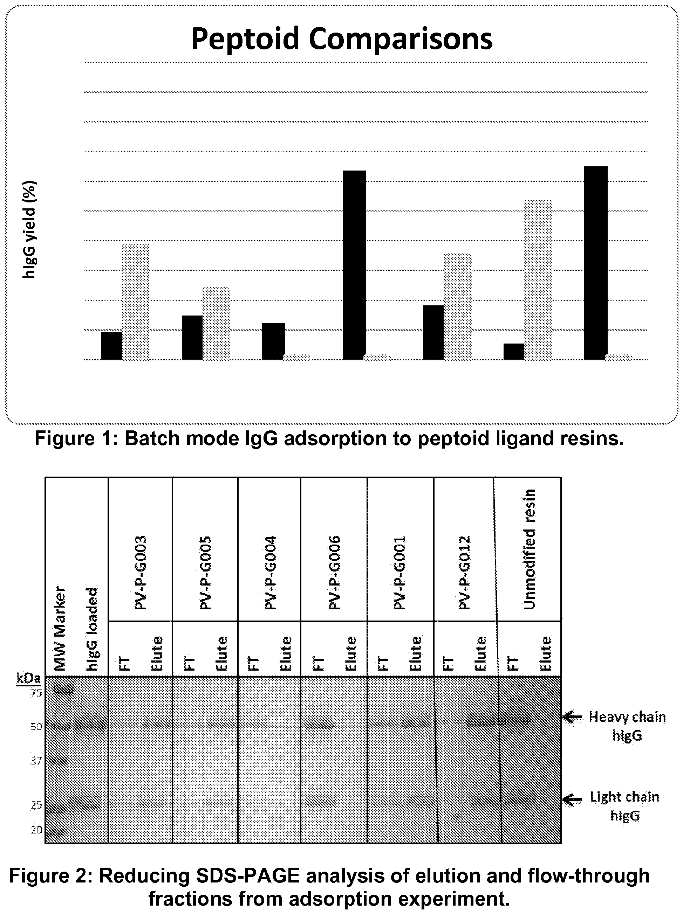

**Figure 1****.** Batch mode IgG adsorption to Toyopearl AF-amino 650M resins containing various peptoid ligands. IgG in the flow-through fractions (black) indicate the percent hlgG that was not adsorbed to the resin relative to the hlgG mass that was loaded. The elution fractions (grey) indicate the percent of human IgG that was bound in phosphate buffered saline (PBS) and eluted (0.1M glycine, pH 2.5) relative to the initial concentration of IgG that was loaded. Concentrations of hlgG in all fractions were determined by ELISA. The unmodified resin served as the negative control. Binding trials were done in duplicate for each resin.
**Figure 2****.** Reducing SDS-PAGE analysis of elution and flow-through fractions from hlgG adsorption experiments. Lanes designated "FT" and "Elute" are the flow-through and elution fractions, respectively. The lane marked "hlgG loaded" is the pre-adsorbed hlgG solution that was incubated with each resin tested. The heavy and light chains of hlgG migrate at ~55 and ~25 kDa, respectively, on reducing SDS-PAGE gels. Bands observed in the flow-through represent hlgG that did not bind the resin, while bands observed in the elute represent hlgG that was adsorbed and subsequently eluted at low pH.

### Detailed Description of Illustrative Embodiments

The present invention now will be described more fully hereinafter with reference to the accompanying figures, in which embodiments of the invention are shown. This invention may, however, be embodied in many alternate forms and should not be construed as limited to the embodiments set forth herein. Like numbers refer to like elements throughout the description of the figures.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the present application and relevant art and should not be interpreted in an idealized or overly format sense unless expressly so defined herein. The terminology used in the description of the invention herein is for the purpose of describing particular embodiments only and is not intended to be limiting of the invention. All publications, patent applications, patents and other references mentioned herein are incorporated by reference in their entirety.

Also as used herein, "and/or" refers to and encompasses any and all possible combinations of one or more of the associated listed items, as well as the lack of combinations when interpreted in the alternative ("or").

### 1. Definitions.

Functional group as used herein may be any suitable group or substituent, including but not limited to H, linear and cyclic alkyl, alkenyl, and alkynyl, possibly substituted and / or functionalized with groups such as alkoxy, halo, mercapto, azido, cyano, formyl, carboxyl, hydroxyl, nitro, acyl, aryloxy, alkylthio, amino, alkylamino, arylalkylamino, substituted amino, acylamino, acyloxy, ester, thioester, carboxylic thioester, ether, amide, amidino, sulfate, sulfoxyl, sulfonyl, sulfonyl, sulfonic acid, sulfonamide, urea, alkoxylacylamino, aminoacyloxy, guanidino, aldehyde, keto, imine, nitrile, phosphate, thiol, epoxide, peroxide, thiocyanate, amidine, oxime, nitrile, diazo, etc., these terms including combinations of these groups (e.g. alkylated groups) as discussed further below.

"Alkyl" as used herein alone or as part of another group, refers to a straight, branched chain, or cyclic, saturated or unsaturated, hydrocarbon containing from 1 or 2 to 10 or 20 carbon atoms, or more. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, and the like. "Lower alkyl" as used herein, is a subset of alkyl, in some embodiments preferred, and refers to a straight or branched chain hydrocarbon group containing from 1 to 4 carbon atoms. Representative examples of lower alkyl include, but are not limited to, methyl, ethyl, n-propyl, iso-propyl, n-butyl, iso-butyl, tert-butyl, and the like. The term "akyl" or "loweralkyl" is intended to include both substituted and unsubstituted alkyl or loweralkyl unless otherwise indicated and these groups may be substituted with groups selected from halo (e.g., haloalkyl), alkyl, haloalkyl, alkenyl, alkynyl, cycloalkyl, cycloalkylalkyl, aryl, arylalkyl, heterocyclo, heterocycloalkyl, hydroxyl, alkoxy (thereby creating a polyalkoxy such as polyethylene glycol), alkenyloxy, alkynyloxy, haloalkoxy, cycloalkoxy, cycloalkylalkyloxy, aryloxy, arylalkyloxy, heterocyclooxy, heterocyclolalkyloxy, mercapto, alkyl-S(O)ₘ, haloalkyl-S(O)ₘ, alkenyl-S(O)ₘ, alkynyl-S(O)ₘ, cycloalkyl-S(O)ₘ, cycloalkylalkyl-S(O)ₘ, aryl-S(O)ₘ, arylalkyl-S(O)ₘ, heterocyclo-S(O)ₘ, heterocycloalkyl-S(O)ₘ, amino, carboxy, alkylamino, alkenylamino, alkynylamino, haloalkylamino, cycloalkylamino, cycloalkylalkylamino, arylamino, arylalkylamino, heterocycloamino, heterocycloalkylamino, disubstituted-amino, acylamino, acyloxy, ester, amide, sulfonamide, urea, alkoxyacylamino, aminoacyloxy, nitro or cyano where m= 0, 1, 2 or 3. Alkyl may be saturated or unsaturated and hence the term "alkyl" as used herein is inclusive of alkenyl and alkynyl when the alkyl substituent contains one or more unsaturated bond (for example, one or two double or triple bonds). The alkyl group may optionally contain one or more heteroatoms (*e.g*., one, two, or three or more heteroatoms independently selected from O, S, and NR', where R' is any suitable substituent such as described immediately above for alkyl substituents), to form a linear heteroalkyl or heterocyclic group as specifically described below.

"Alkenyl" as used herein refers to an alkyl group as described above containing at least one double bond between two carbon atoms therein.

"Alkynyl" as used herein refers to an alkyl group as described above containing at least one triple bond between two carbon atoms therein.

"Alkylene" as used herein refers to an alkyl group as described above, with one terminal hydrogen removed to form a bivalent substituent.

"Heterocyclic group" or "heterocyclo" as used herein alone or as part of another group, refers to an aliphatic (e.g., fully or partially saturated heterocyclo) or aromatic (e.g., heteroaryl) monocyclic- or a bicyclic-ring system. Monocyclic ring systems are exemplified by any 5 or 6 membered ring containing 1, 2, 3, or 4 heteroatoms independently selected from oxygen, nitrogen and sulfur. The 5 membered ring has from 0-2 double bonds and the 6 membered ring has from 0-3 double bonds. Representative examples of monocyclic ring systems include, but are not limited to, azetidine, azepine, aziridine, diazepine, 1,3-dioxolane, dioxane, dithiane, furan, imidazole, imidazoline, imidazolidine, isothiazole, isothiazoline, isothiazolidine, isoxazole, isoxazoline, isoxazolidine, morpholine, oxadiazole, oxadiazoline, oxadiazolidine, oxazole, oxazoline, oxazolidine, piperazine, piperidine, pyran, pyrazine, pyrazole, pyrazoline, pyrazolidine, pyridine, pyrimidine, pyridazine, pyrrole, pyrroline, pyrrolidine, tetrahydrofuran, tetrahydrothiophene, tetrazine, tetrazole, thiadiazole, thiadiazoline, thiadiazolidine, thiazole, thiazoline, thiazolidine, thiophene, thiomorpholine, thiomorpholine sulfone, thiopyran, triazine, triazole, trithiane, and the like. Bicyclic ring systems are exemplified by any of the above monocyclic ring systems fused to an aryl group as defined herein, a cycloalkyl group as defined herein, or another monocyclic ring system as defined herein. Representative examples of bicyclic ring systems include but are not limited to, for example, benzimidazole, benzothiazole, benzothiadiazole, benzothiophene, benzoxadiazole, benzoxazole, benzofuran, benzopyran, benzothiopyran, benzodioxine, 1,3-benzodioxoie, cinnoline, indazole, indole, indoline, indolizine, naphthyridine, isobenzofuran, isobenzothiophene, isoindole, isoindoline, isoquinoline, phthalazine, purine, pyranopyridine, quinotine, quinolizine, quinoxaline, quinazoline, tetrahydroisoquinoline, tetrahydroquinoline, thiopyranopyridine, and the like. These rings include quaternized derivatives thereof and may be optionally substituted with groups selected from halo, alkyl, haloalkyl, alkenyl, alkynyl, cycloalkyl, cycloalkylalkyl, aryl, arylalkyl, heterocyclo, heterocycloalkyl, hydroxyl, alkoxy, alkenyloxy, alkynyloxy, haloalkoxy, cycloalkoxy, cycloalkylalkyloxy, aryloxy, arylalkyloxy, heterocyclooxy, heterocyclolalkyloxy, mercapto, alkyl-S(O)ₘ, haloalkyl-S(O)ₘ, alkenyl-S(O)ₘ, alkynyl-S(O)ₘ, cycloalkyl-S(O)ₘ, cycloalkylalkyl-S(O)ₘ, aryl-S(O)ₘ, arylalkyl-S(O)ₘ, heterocyclo-S(O)ₘ, heterocycloalkyl-S(O)ₘ, amino, alkylamino, alkenylamino, alkynylamino, haloalkylamino, cycloalkylamino, cycloalkylalkylamino, arylamino, arylalkylamino, heterocycloamino, heterocycloalkylamino, disubstituted-amino, acylamino, acyloxy, ester, amide, sulfonamide, urea, alkoxyacylamino, aminoacyloxy, nitro or cyano wherem=0, 1, 2 or 3.

"Aryl" as used herein alone or as part of another group, refers to a monocyclic carbocyclic ring system or a bicyclic carbocyclic fused ring system having one or more aromatic rings. Representative examples of aryl include, azulenyl, indanyl, indenyl, naphthyl, phenyl, tetrahydronaphthyl, and the like. The term "aryl" is intended to include both substituted and unsubstituted aryl unless otherwise indicated and these groups may be substituted with the same groups as set forth in connection with alkyl and loweralkyl above.

"Arylalkyl" as used herein alone or as part of another group, refers to an aryl group, as defined herein, appended to the parent molecular moiety through an alkyl group, as defined herein. Representative examples of arylalkyl include, but are not limited to, benzyl, 2-phenylethyl, 3-phenylpropyl, 2-naphth-2-ylethyl, and the like.

"Heteroaryl" as used herein is as described in connection with heterocyclo above.

"Alkoxy" as used herein alone or as part of another group, refers to an alkyl or loweralkyl group, as defined herein (and thus including substituted versions such as polyalkoxy), appended to the parent molecular moiety through an oxy group, -O-. Representative examples of alkoxy include, but are not limited to, methoxy, ethoxy, propoxy, 2-propoxy, butoxy, tert-butoxy, pentyloxy, hexyloxy and the like.

"Halo" as used herein refers to any suitable halogen, including -F, -Cl, -Br, and -I.

"Acyl" as used herein alone or as part of another group refers to a -C(O)R radical, where R is any suitable substituent such as aryl, alkyl, alkenyl, alkynyl, cycloalkyl or other suitable substituent as described herein.

"Alkylthio" as used herein alone or as part of another group, refers to an alkyl group, as defined herein, appended to the parent molecular moiety through a thio moiety, as defined herein. Representative examples of alkylthio include, but are not limited, methylthio, ethylthio, tert-butylthio, hexylthio, and the like.

"Alkylamino" as used herein alone or as part of another group means the radical - NHR, where R is an alkyl group.

"Arylalkylamino" as used herein alone or as part of another group means the radical -NHR, where R is an arylalkyl group.

"Disubstituted-amino" as used herein alone or as part of another group means the radical -NRₐR_{b}, where Rₐ and R_{b} are independently selected from the groups alkyl, haloalkyl, alkenyl, alkynyl, cycloalkyl, cycloalkylalkyl, aryl, arylalkyl, heterocyclo, heterocycloalkyl,

"Acylamino" as used herein alone or as part of another group means the radical - NRₐR_{b}, where Rₐ is an acyl group as defined herein and R_{b} is selected from the groups hydrogen, alkyl, haloalkyl, alkenyl, alkynyl, cycloalkyl, cycloalkylalkyl, aryl, arylalkyl, heterocyclo, heterocycloalkyl.

"Acyloxy" as used herein alone or as part of another group means the radical -OR, where R is an acyl group as defined herein.

"Ester" as used herein alone or as part of another group refers to a -C(O)OR radical, where R is any suitable substituent such as alkyl, cycloalkyl, alkenyl, alkynyl or aryl.

"Amide" as used herein alone or as part of another group refers to a -C(O)NRₐR_{b} radical or a -N(Rₐ)C(O)R_{b} radical, where Rₐ and R_{b} are any suitable substituent such as alkyl, cycloalkyl, alkenyl, alkynyl or aryl.

"Sulfoxyl" as used herein refers to a compound of the formula -S(O)R, where R is any suitable substituent such as alkyl, cycloalkyl, alkenyl, alkynyl or aryl.

"Sulfonyl" as used herein refers to a compound of the formula -S(O)(O)R, where R is any suitable substituent such as alkyl, cycloalkyl, alkenyl, alkynyl or aryl.

"Sulfonate" as used herein refers to a compounnd of the formula -S(O)(O)OR, where R is any suitable substituent such as alkyl, cycloalkyl, alkenyl, alkynyl or aryl.

"Sulfonic acid" as used herein refers to a compound of the formula -S(O)(O)OH,

"Sulfonamide" as used herein alone or as part of another group refers to a - S(O)₂NRₐR_{b} radical, where Rₐ and R_{b} are any suitable substituent such as H, alkyl, cycloalkyl, alkenyl, alkynyl or aryl.

"Urea" as used herein alone or as part of another group refers to an - N(R_{c})C(O)NRₐR_{b} radical, where Rₐ, R_{b} and R_{c} are any suitable substituent such as H, alkyl, cycloalkyl, alkenyl, alkynyl or aryl.

"Alkoxyacylamino" as used herein alone or as part of another group refers to an - N(Rₐ)C(O)OR_{b} radical, where Rₐ, R_{b} are any suitable substituent such as H, alkyl, cycloalkyl, alkenyl, alkynyl or aryl.

"Aminoacyloxy" as used herein alone or as part of another group refers to an - OC(O)NRₐR_{b} radical, where Rₐ and R_{b} are any suitable substituent such as H, alkyl, cycloalkyl, alkenyl, alkynyl or aryl.

"Solid support" as used herein may comprise any suitable material, including organic materials (*e.g.*, organic polymers), metals (*e.g*., titanium), inorganic materials (*e.g*., silica) and composites thereof. The solid supports may be in any suitable shape or form, including films, receptacles such as microtiter plate wells (*e.g*., floors and/or walls thereof) particles (*e.g*., beads formed from natural or synthetic polymers, inorganic materials such as glass or silica, composites thereof, etc.) such as for chromatography column packings, etc.

"Coupling group" as used herein may be any suitable reactive group, *e.g*., an alkene, alkyne, alcohol, thiol, selenyl, phosphono, carboxylic acid, formyl, halide or amine group, displayed directly by the parent molecule or by means of an intervening linker group (*e.g*., an aliphatic, aromatic, or mixed aliphatic/aromatic group such as an alkyl, aryl, arylalkyl, or alkylarylalkyl group, etc.).

### 2. Compounds.

Compounds useful for carrying out the present invention include compounds of **Formulas I-IV:** 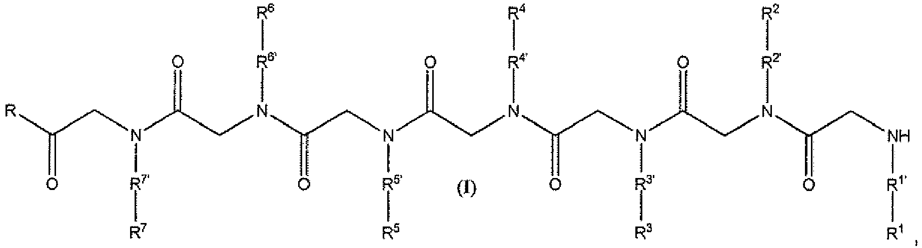 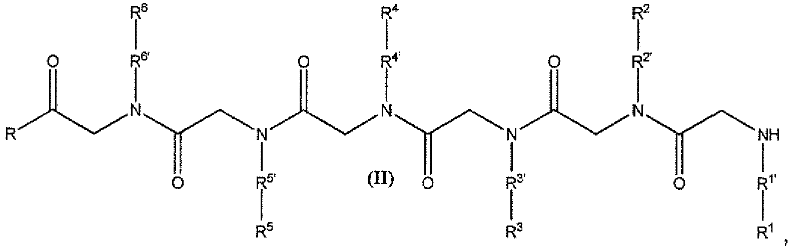 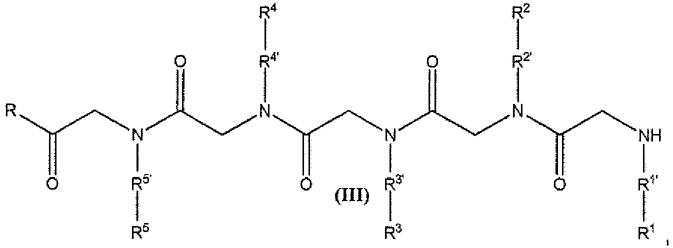 and 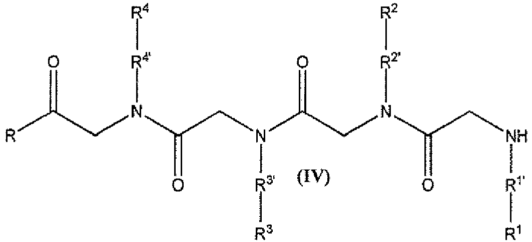 wherein:
R is -OH, -NH₂, -OR', or -NHR', where R' is a solid support;
R^{1'}, R^{2'}, R^{3'}, R^{4'}, R^{5'}, R^{6'}, and R^{7'} are each independently a C1 to C4 alkylene group;
R¹ is a basic group, such as a basic aromatic group, examples of which include but are not limited to pyridinyl-, quinolinyl- and isoquinolinyl-, acridinyl-, pyrazinyl-, quinoxalinyl-, imidazolyl-, benzimidazolyl-, purinyl-, pyrazolyl-, indazolyl-, pyrimidinyl-, quinazolinyl-, pyridazinyl-, cinnolinyl-, and the like;
R² is an aromatic group, examples of which include but are not limited to *(a)* non-substituted, *e.g.* phenyl-, benzyl-, naphtalyl-, and related groups or derivatives thereof; or (b) substituted, *e.g*. indolyl-, and related groups or derivatives thereof; and *(c)* derivatives of the foregoing obtained by functionalization, *e.g*. hydroxylation, amination, carboxylation, and the like, alkylation, alkenylation, alkinylation, and the like, and derivatives thereof;
R³ is a basic group, such as a basic aliphatic group, examples of which include but are not limited to comprising but not limited to the group of primary, secondary, tertiary and quaternary amine, guanidinyl-, and related groups; or an hydrophilic moiety capable of forming hydrogen bonds, *e.g.* carbamoyl-, hydroxyl-, urea, and the like;
R⁴ is H (or also for compounds of **Formula IV**, an aliphatic group, an aromatic group, an acidic group, a hydrophilic group, a basic group, or coupling group).
R⁵ is an aromatic group, a basic group, or a basic aromatic group, such as defined for R¹ and R² above (or also for compounds of **Formula III**, an aliphatic group, an an acidic group, a hydrophilic group, or coupling group),
R⁶ is H, or an acidic group such as a carboxyl group, a sulfonate group, etc (or also for compounds of **Formula II**, an aliphatic group, an aromatic group, a hydrophilic group, a basic group, or coupling group);
R⁷ is H or any of the groups given in connection with R¹ through R⁶ above, or a coupling group group suitable for covalently coupling the peptoid ligand with another (a) natural, *e.g*. a protein, a polynucleotide, a lipid, a peptide, and derivatives thereof, or (b) synthetic compound, *e.g*. a drug, an organic compound, a metallorganic compound, an inorganic compound, or (c) a surface, *e.g.* a chromatographic resin, a membrane, a metal surface, a fiber, a self-assembled structure, or derivatives thereof.

In some embodiments the solid support comprises a particle (*e.g.*, a porous polymer bead like a pearl of chromatographic resin).

In some embodiments the solid support comprises an inorganic material (*e.g.*, silica, titania, zirconia, and the like).

In some embodiments, the solid support comprises an organic polymer material (*e.g*., polyethersulfone, PMMA, etc.).

Peptoid compounds of the present invention such as compounds of **Formulas I-IV** can be prepared in accordance with known techniques, including but not limited to those descrbed in: N.J. Brown, J. Johansson, and A.E. Barron, Acc. Chem. Res. 41, 1409-1417 (2008); N.P. Chongsiriwatana, J.A. Patch, A.M. Czyzewski, M.T. Dohm, A. Ivankin, D. Gidalevitz, R.N. Zuckermann, and A.E. Barron, PNAS 105, 2794-2799 (2008); K.E. Drexler, Peptide Science 96, 537-544 (2011); B.C. Gorske, B.L. Bastian, G.D. Geske, and H.E. Blackwell, J. Am. Chem. Soc. 129, 8928-8929 (2007); T. Hara, S.R. Durell, M.C. Myers, and D.H. Appella, J. Am. Chem. Soc. 128, 1995-2004 (2006); R.D. Haynes, R.J. Meagher, J.-I. Won, F.M. Bogdan, and A.E. Barron, Bioconjugate Chem. 16, 929-938 (2005); K. Kirshenbaum, A.E. Barron, R.A. Goldsmith, P. Armand, E.K. Bradley, K.T.V. Truong, K.A. Dill, F.E. Cohen, and R.N. Zuckermann, PNAS 95, 4303-4308 (1998); Y.-U. Kwon and T. Kodadek, J. Am. Chem. Soc. 129, 1508-1509 (2007); G. Maayan, M.D. Ward, and K. Kirshenbaum, PNAS 106, 13679-13684 (2009); S.M. Miller, R.J. Simon, S. Ng, R.N. Zuckermann, J.M. Kerr, and W.H. Moos, Drug Development Research 35, 20-32 (1995); P. Mora, I. Masip, N. Cortés, R. Marquina, R. Merino, J. Merino, T. Carbonell, I. Mingarro, A. Messeguer, and E. Pérez-Payá, J. Med. Chem. 48, 1265-1268 (2005); J.E. Murphy, T. Uno, J.D. Hamer, F.E. Cohen, V. Dwarki, and R.N. Zuckermann, PNAS 95, 1517-1522 (1998); K.T. Nam, S.A. Shelby, P.H. Choi, A.B. Marciel, R. Chen, L. Tan, T.K. Chu, R.A. Mesch, B.-C. Lee, M.D. Connolly, C. Kisielowski, and R.N. Zuckermann, Nature Materials 9, 454-460 (2010); J.T. Nguyen, M. Porter, M. Amoui, W.T. Miller, R.N. Zuckermann, and W.A. Lim, Chem. Biol. 7, 463-473 (2000); P.E. Nielsen, ed., Pseudo-peptides in Drug Discovery, 1 st ed. (Wiley-VCH, 2004); S.H. Park and I. Szleifer, J. Phys. Chem. B 115, 10967-10975 (2011); J.A. Patch and A.E. Barron, J. Am. Chem. Soc. 125, 12092-12093 (2003); I. Peretto, R.M. Sanchez-Martin, X. Wang, J. Ellard, S. Mittoo, and M. Bradley, Chem. Commun. 2312-2313 (n.d.); M.C. Pirrung, K. Park, and L.N. Tumey, J. Comb. Chem. 4, 329-344 (2002); M.M. Reddy and T. Kodadek, Proc. Nat. Acad. Sci. USA 102, 12672-12677 (2005); M.M. Reddy, R. Wilson, J. Wilson, S. Connell, A. Gocke, L. Hynan, D. German, and T. Kodadek, Cell 144, 132-142 (2011); T.J. Sanborn, C.W. Wu, R.N. Zuckermann, and A.E. Barron, Biopolymers 63, 12-20 (2002); T. Schröder, N. Niemeier, S. Afonin, A.S. Ulrich, H.F. Krug, and S. Bräse, J. Med. Chem. 51, 376-379 (2008); N.H. Shah, G.L. Butterfoss, K. Nguyen, B. Yoo, R. Bonneau, D.L. Rabenstein, and K. Kirshenbaum, J. Am. Chem. Soc. 130, 16622-16632 (2008); A. Statz, J. Kuang, C. Ren, A. Barron, I. Szleifer, and P. Messersmith, Biointerphases 4, FA22-FA32 (2009); A.R. Statz, J.P. Park, N.P. Chongsiriwatana, A.E. Barron, and P.B. Messersmith, Biofouling 24, 439-448 (2008); P.A. Wender, D.J. Mitchell, K. Pattabiraman, E.T. Pelkey, L. Steinman, and J.B. Rothbard, Proc. Nat. Acad. Sci. USA 97, 13003-13008 (2000); C.W. Wu, S.L. Seurynck, K.Y.C. Lee, and A.E. Barron, Chem. Biol. 10, 1057-1063 (2003); R.N. Zuckermann, J.M. Kerr, S.B.H. Kent, and W.H. Moos, J. Am. Chem. Soc. 114, 10646-10647 (1992); R. N. Zuckermann, E.J. Martin, D.C. Spellmeyer, G.B. Sfauber, K.R. Shoemaker, J.M. Kerr, G.M. Figliozzi, D.A. Goff, and M. A. Siani, J. Med. Chem. 37, 2678-2685 (1994*)*.

### B. Methods of use.

The peptoids of the present invention may be used to bind to, collect, purify, etc., any type of antibody or antibody fragment (*e.g*., Fc fragments, Fab fragments, and scFV fragments), including both natural and recombinant (including chimeric) antibodies, engineered multibodies, single domain antibodies, and combinations thereof, such as divalent antibodies and camelid immunoglobulins, and both monoclonal and polyclonal antibodies, or an Fc-fusion protein. The antibodies may be of any species of origin, including mammalian (rabbit, mouse, rat, cow, goat, sheep, Ilama, camel, alpaca, etc), avian (*e.g.*, chicken, turkey, etc.), shark, etc., including fragments, chimeras and combinations thereof as noted above.The antibodies may be of any type of immunoglobulin, including but not limited to IgG, IgA, IgE, IgD, IgM, IgY, etc.

In some embodiments the antibodies or Fc fragments (including fusion proteins thereof) are carried in a biological fluid such as blood or a blood fraction (*e.g*., blood sera, blood plasma), egg yolk and/or albumin, tissue or cell growth media, a tissue lysate or homogenate, etc.

Thus, as noted above, the present invention provides a method of binding an antibody or antibody Fc fragment from a liquid composition containing the same, comprising the steps of:
(a) providing a solid support comprising a compound as described above
(b) contacting said composition to said solid support so that antibody or Fc fragment or Fc-fusion protein bind to said compound; and
(c) separating said liquid composition from said solid support, with said antibody or Fc fragment or Fc-fusion protein bound to said solid support; and optionally (but in some embodiments preferably)
(d) separating said antibody or Fc fragment or Fc-fusion protein from said solid support.

The methods can be carried out in like manner to those employing protein A, or by variations thereof that will be apparent to those skilled in the art. For example, the contacting and separating steps can be carried out continuously, (*e.g*., by column chromatography), after which the separating step can then be carried out (*e.g*., by elution), in accordance with known techniques.

In some embodiments, such as when the liquid composition from which the antibodies or Fc fragments or Fc-fusion proteins are to be collected, comprises a biological fluid, the liquid composition further comprises at least one proteolytic enzyme. The peptoid binding ligands are advantageously resistant to degredation by the proteolytic enzymes.

In addition, photoaffinity labeling of all the above mentioned target antibodies can be carried out by replacing any of the side-chain residues of the peptoid with a photoreactive group such as a benzophenone group.

The present invention is explained in greater detail in the following nonlimiting Examples.

### EXAMPLE 1

### Solid-phase Synthesis of a Tetramer Peptoid Ligand and Use of the Resulting Affinity Adsorbent for the Purification of Human Polyclonal or Monoclonal Antibodies

The selected groups, as numbered from the N- to the C- terminus, are: imidazoyl, indolyl-, guanidyl-, and methyl-. Correspondingly, the amines employed for the peptoid synthesis are respectively histamine, tryptamine, agmatine, and methylamine. Presented in Scheme 1, as follows:

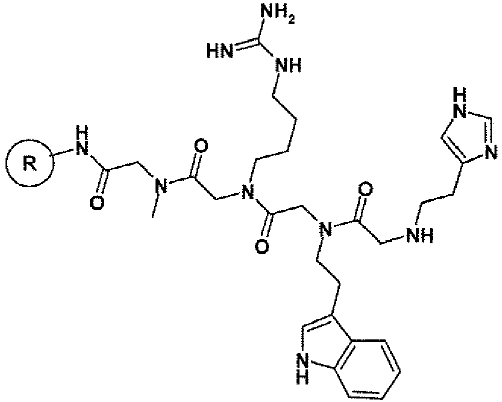

The chromatographic resin Toyopearl AF-Amino-650M is chosen as solid support for synthesis. The synthesis comprises the following steps:
1. Coupling of bromoacetic acid in N,N'-dimethylformamide (DMF) via diisopropylcarbodiimide activation (hereafter referred to as DIC). Wash the resin with DMF and equilibrate with NMP.
2. Coupling of methylamine in N-methylpyrrolidone (NMP). Wash the resin with NMP and equilibrate with DMF.
3. Coupling of bromoacetic acid by DIC. Wash the resin with DMF and equilibrate with NMP.
4. Coupling of agmatine. Wash the resin with NMP and equilibrate with DMF.
5. Coupling of bromoacetic acid by DIC. Wash the resin with DMF and equilibrate with NMP.
6. Coupling of Nᵢₙ-Boc-tryptamine. Wash the resin with NMP and equilibrate with DMF.
7. Coupling of bromoacetic acid by DIC. Wash the resin with DMF and equilibrate with NMP.
8. Coupling of Nᵢₘ-Boc-histamine. Wash the resin with NMP, followed by DMF, and finally equilibrate the resin with DCM. Remove the Boc protecting groups by washing the resin two times with 75% TFA (trifluoroacetic acid) in DCM for 45min, at room temperature. Wash the resin sequentially with DCM, DMF and DCM.

The resin is then vacuum dried, packed in a chromatographic column, swollen in 20% v/v methanol in water, and equilibrated in phosphate buffer saline (PBS), pH 7.4.

A solution of human polyclonal IgG at 5 mg/mL in PBS is prepared and injected in the column at the linear velocity of 67 cm/h. After washing the column with 10 column volumns of equilibration buffer at the linear velocity of 200 cm/h, the bound antibody is eluted with 0.2M Acetate buffer pH 4 at the linear velocity of 270cm/h. The resin is finally regenerated with aqueous 0.1 M NaOH.

### EXAMPLE 2

### Preparation of Peptoids Directly to Amine-Containing Resin

**Preparation Procedures**: Peptoids were synthesized directly onto Toyopearl AF-amino 650M resin (Tosoh Biosciences) at a 0.1 mmole/mL loading with a Biotage Alstra automated peptide synthesizer under microwave assistance using methods described previously (Fara et al. Tet. Lett. 2006 47, 1011-1014; Olivos et al. Org. Lett. 2002, 4(23), 4057-4059). The FMOC-protected monomers for glycine, N-(-3-guanidinpropyl)glycine, N-(isobutyl)glycine, N-(3-(Boc-amino)-propyl)glycine and N-(benzyl)glycine were coupled to the peptoid using *N,N,N',N'*-Tetramethyl-*O*-(1*H*-benzotriazol-1-yl)uronium hexafluorophosphate (HBTU) with diisopropylethyl amine as the coupling agent in dimethyl formamide for 30 minutes at 50C (Huang et al. PNAS 2012, 109(49), 19922-19927.). After coupling, the secondary amine was deprotected using 20% piperidine in DMF. The remaining residues were coupled to the peptoid sequence using a submonomer approach. Chloroacetic acid was coupled to the previous residue with diisopropylcarbodiimide (15 minutes, 75C) followed by displacement of the chloride with the corresponding amine and potassium iodide as catalyst (1 hour, 50 C). The following amines were used in the halogen displacement step: 2-propylamine, histamine, and tryptamine (Zuckerman et al. J. AM. CHEM. SOC. 2003,125, 8841). The ligands were deprotected directly on the resin using 90% trifluoroacetic acid, 5% triisopropylsilane, and 5% H₂O, followed by successive washing with triflouroacetic acid, methanol, and water.

Substituents on the immobilized peptoids are as given in **Table 1** below, where the various "R" groups correspond to the **Formulas** above. The structure of the peptoids when free from (not covalently coupled to) a resin is given in **Table 2** below.

**TABLE 1**

| ID | PV-P-G001 | PV-P-G003 | PV-P-G004 | PV-P-G005 | PV-P-G006 | PV-P-G012 |
|---|---|---|---|---|---|---|
| R¹ | -2-imidazole | -2-imidazole | -2-imidazole | -2-imidazole | -2-imidazole | -2-imidazole |
| R¹ | -CH₂ CH₂- | -CH₂ CH₂- | -CH₂CH₂- | -CH₂CH₂- | -CH₂CH₂- | -CH₂CH₂- |
| R² | -3-insole | -3-indole | -C₆H₅ | -3-indole | -C₆H₅ | -3-indole |
| R² | -CH₂ CH₂- | -CH₂ CH₂- | -CH₂CH₂- | -CH₂CH₂- | -CH₂- | -CH₂CH₂ |
| R³ | -NH-(C=NH)-NH₂ (guanidyl) | -NH-(C=NH)-NH₂ (guanidyl) | -NH-(C=NH)-NH₂ (guanidyl) | -NH-(C=NH)-NH₂ (guanidyl) | -NH-(C=NH)-NH₂ (guanidyl) | -NH2 |
| R³ | -CH₂CH₂CH₂- | -CH₂CH₂CH₂- | -CH₂CH₂CH₂- | -CH₂CH₂CH₂- | -CH₂CH₂CH₂- | -CH₂CH₂CH₂- |
| R⁴ | H | H | H | | | H |
| R⁴ | N/A | N/A | N/A | | | N/A |
| R⁵ | -CH₂ CH₂- | | | | | -3-indole |
| R⁵ | -3-indole | | | | | -CH₂CH₂- |
| R⁶ | -CH(CH₃)₂ | | | | | -CH(CH₃)₂ |
| R⁶ | N/A | | | | | N/A |

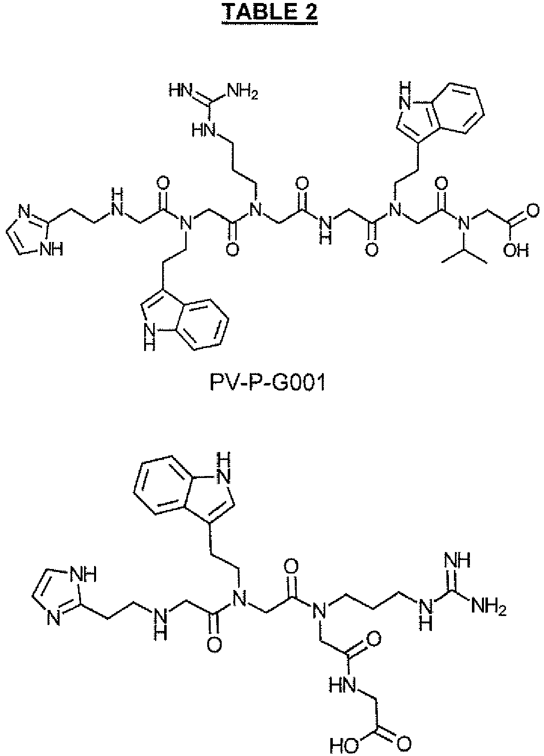

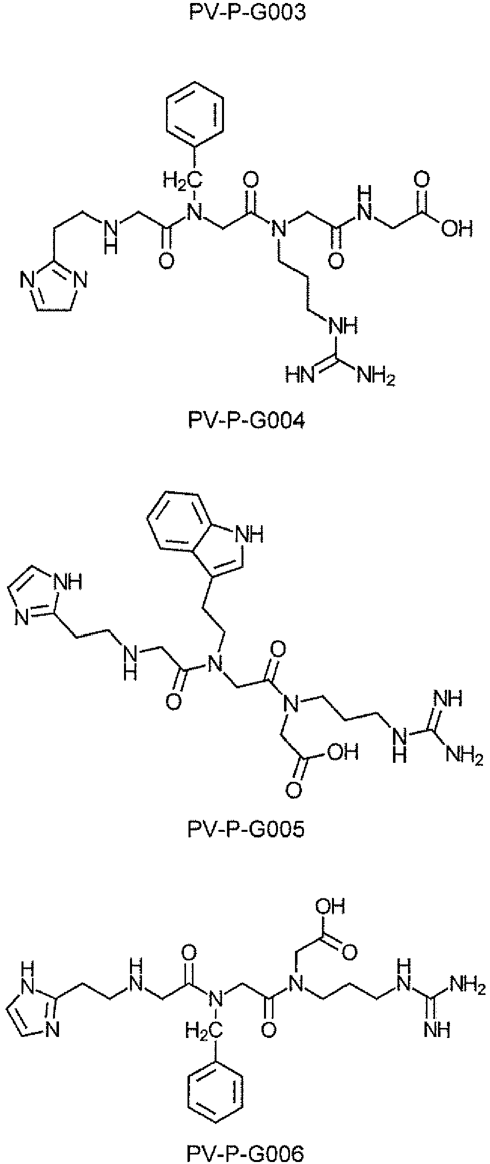

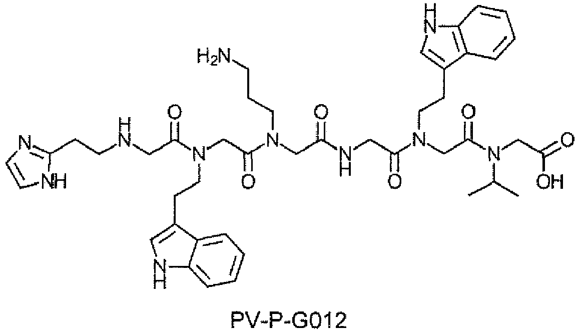

**IgG Binding to Resin:** The ability of the peptoid affinity ligands to bind human IgG molecules was tested in batch mode. A 50% slurry of the resin was prepared in which 200ul (~100mg resin) was transferred into the spin column. The resin was extensively washed with dH2O and equilibrated with PBS prior to hlgG exposure. hlgG (Equitech-Bio) was diluted to 0.5mg/ml with PBS. After equilibration, 0.5mg/ml hlgG was added to the resin at a fixed ratio (2mg hlgG per ml of resin) and allowed to incubate for 45 min at 22°C with vigorous shaking. After incubation the resin was centrifuged at 500 x g for 2 min in which the filtrate of this step was collected and called the "flow-through" fraction. The resin was subsequently washed 2X with PBS. Elution buffer (0.1 M glycine, pH 2.5) was added to the resin and was allowed to mix vigorously for 10 min at 22°C. After centrifugation (500x g for 2 min) bound hlgG was eluted from the resin and collected in the filtrate. The amount of hlgG found in either the flow-through or elution fraction was determined using an ELISA-based quantitative assay (Bethyl, Cat. No. E80-104) Percent hlgG yields were determined by dividing the concentration of hlgG in either the flow-through or elution fraction by the concentration of the loading material (**Figure 1**). SDS-PAGE was also done to analyze the fractions of the adsorption experiments (**Figure 2**). These data indicated that peptoids G001, G003, G005, and G012 were useful for binding IgG to the resin.

## Claims

1. A compound selected from the group consisting of compounds of **Formulas I-IV:** 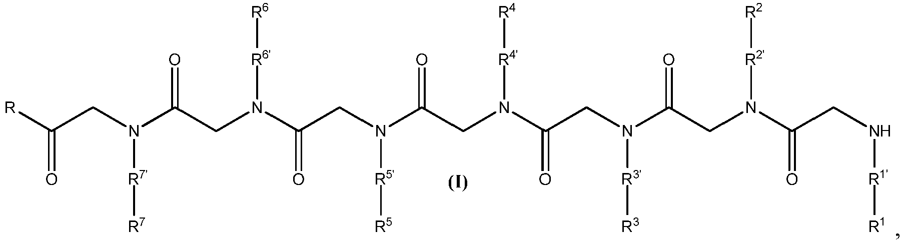 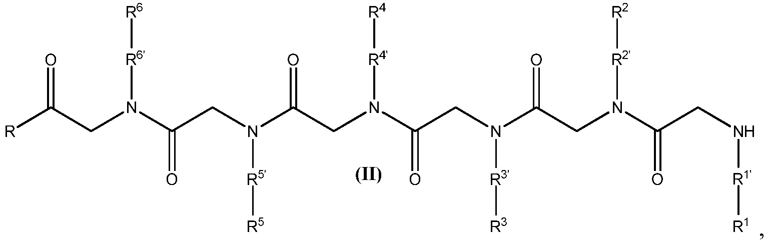 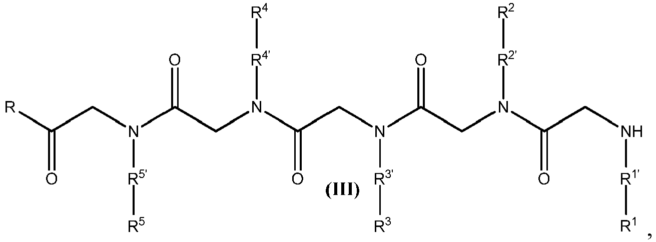 and 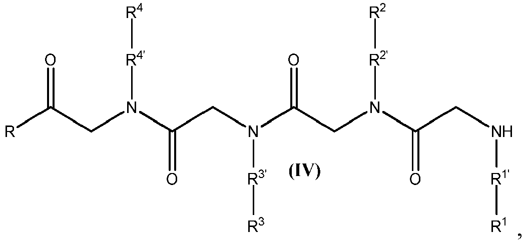 wherein:
R is -OH, -NH₂, -OR', or NHR', where R' is a solid support and the compound is optionally attached to the solid support via a coupling group;
R^{1'}, R^{2'}, R^{3'}, R^{5'} and R^{7'} are each independently a C1 to C4 alkylene group;
R^{4'} and R^{6'} are each independently absent or a C1 to C4 alkylene group;
R¹ is a basic group;
R² is an aromatic group;
R³ is a basic group or a hydrophilic group;
R⁴ is H or also for compounds of **Formula IV,** an aliphatic group, an aromatic group, an acidic group, a hydrophilic group, a basic group, or coupling group;
R⁵ is an aromatic group, a basic group, or also for compounds of **Formula III**, an aliphatic group, an acidic group, a hydrophilic group, or coupling group;
R⁶ is H, an acidic group, or also for compounds of **Formula II**, an aliphatic group, an aromatic group, a hydrophilic group, a basic group, or coupling group;
R⁷ is H, an aliphatic group, an aromatic group, an acidic group, a hydrophilic group a basic group, or a coupling group.

2. A compound of claim 1 having a structure of **Formula I**: 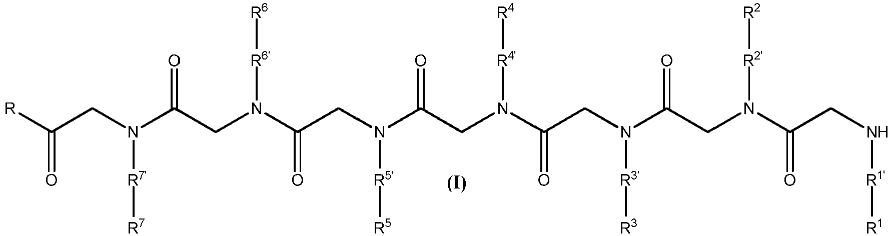 wherein:
R is -OH, -NH₂, -OR', or NHR', where R' is a solid support and the compound is optionally attached to the solid support via a coupling group;
R^{1'}, R^{2'}, R^{3'}, R⁵ and R^{7'} are each independently a C1 to C4 alkylene group;
R^{4'} and R^{6'} are each independently absent or a C1 to C4 alkylene group;
R¹ is a basic group;
R² is an aromatic group;
R³ is a basic group or a hydrophilic group;
R⁴ is H;
R⁵ is an aromatic group or a basic group;
R⁶ is H or an acidic group;
R⁷ is H, an aliphatic group, an aromatic group, an acidic group, a hydrophilic group a basic group, or a coupling group.

3. A compound of claim 1 having the structure of **Formula II**: 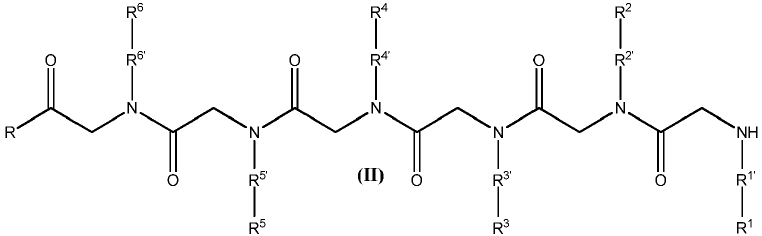 wherein:
R is -OH, -NH₂, -OR', or NHR', where R' is a solid support and the compound is optionally attached to the solid support via a coupling group;
R^{1'}, R^{2'}, R^{3'}, R^{5'}, and R^{7'} are each independently a C1 to C4 alkylene group;
R^{4'} and R^{6'} are each independently absent or a C1 to C4 alkylene group;
R¹ is a basic group;
R² is an aromatic group;
R³ is a basic group or a hydrophilic group;
R⁴ is H;
R⁵ is an aromatic group or a basic group;
R⁶ is H, an acidic group, an aliphatic group, an aromatic group, a hydrophilic group, a basic group, or a coupling group.

4. A compound of claim 1 having the structure of **Formula III**: 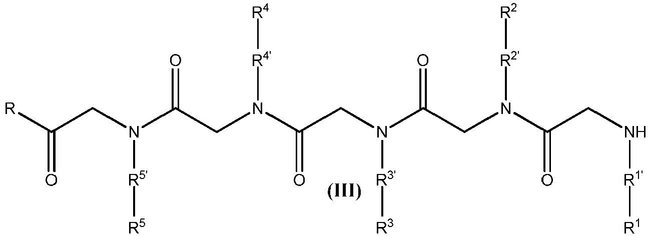 wherein:
R is -OH, -NH₂, -OR', or NHR', where R' is a solid support and the compound is optionally attached to the solid support via a coupling group;
R^{1'}, R^{2'}, R^{3'}, R^{5'}, and R^{7'} are each independently a C1 to C4 alkylene group;
R^{4'} and R^{6'} are each independently absent or a C1 to C4 alkylene group;
R¹ is a basic group;
R² is an aromatic group;
R³ is a basic group or a hydrophilic group;
R⁴ is H;
R⁵ is an aromatic group, a basic group, an aliphatic group, an acidic group, a hydrophilic group, or coupling group.

5. A compound of claim 1 having the structure of **Formula IV:** 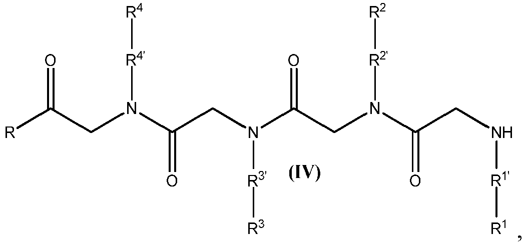 wherein:
R is -OH, -NH₂, -OR', or NHR', where R' is a solid support and the compound is optionally attached to the solid support via a coupling group;
R^{1'}, R^{2'}, R^{3'}, R⁵, and R^{7'} are each independently a C1 to C4 alkylene group;
R^{4'} and R^{6'} are each independently absent or a C1 to C4 alkylene group;
R¹ is a basic group;
R² is an aromatic group;
R³ is a basic group or a hydrophilic group;
R⁴ is H, an aliphatic group, an aromatic group, an acidic group, a hydrophilic group, a basic group, or a coupling group.

6. The compound of claim 1, wherein:
R¹ is a basic aromatic group; and/or
R⁵ is a basic aromatic group.

7. The compound of claim 1, wherein:
R¹ is selected from the group consisting pyridinyl-, quinolinyl-, isoquinolinyl-, acridinyl-, pyrazinyl-, quinoxalinyl-, imidazolyl-, benzimidazolyl-, purinyl-, pyrazolyl-, indazolyl-, pyrimidinyl-, quinazolinyl-, pyridazinyl-, and cinnolinyl-; and/or
R² is selected from the group consisting of phenyl-, benzyl-, naphtalyl-, indolyl-, and substituted derivatives thereof; and/or
R³ is selected from the group consisting of primary, secondary, tertiary and quaternary amine, guanidinyl-, carbamoyl-, hydroxyl-, and urea; and/or
R⁴ is H, and/or
R⁵ is an aromatic group, a basic group, or a basic aromatic group.

8. The compound of claim 1 to 7, wherein said solid support comprises a particle.

9. The compound of claim 1 to 8, wherein said solid support comprises an inorganic material, or
wherein said solid support comprises an organic polymer material.

10. A method of binding an antibody or antibody Fc fragment from a liquid composition containing the same, comprising the steps of:
(a) providing a solid support comprising a compound of claim 1 to 9, wherein R is -OR' or NHR' and the compound is optionally attached to said solid support via a coupling group;
(b) contacting said composition to said solid support so that said antibody or Fc fragments bind to said compound; and
(c) separating said liquid composition from said solid support, with said antibody or Fc fragment bound to said solid support.

11. The method of claim 10, wherein said liquid composition further comprises at least one proteolytic enzyme and/or said liquid composition further comprises a biological fluid, optionally wherein said biological fluid comprises blood, blood sera, blood plasma, tissue or cell culture media, a cell lysate, a plant extract, or a fluid produced and/or secreted by a recombinant animal.

12. The method of any one of claims 10 to 11, wherein said contacting and separating steps are carried out continuously.

13. The method of any one of claims 10 to 12, further comprising the step of:
(d) separating said antibody or antibody Fc fragment from said solid support.

14. The method of any one of claims 10 to 13, wherein the antibody or antibody Fc fragment is a IgG, IgA, IgE, IgD, IgM, or IgY antibody or antibody fragment, optionally wherein the antibody or antibody Fc fragment is a mammalian antibody or antibody fragment.

15. The compound of claim 1-9 or method of claim 10 to 14, wherein said compound is selected from the group consisting of:

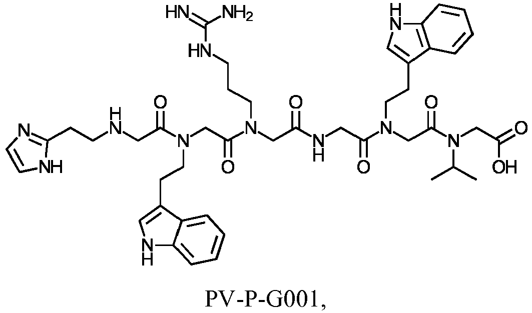

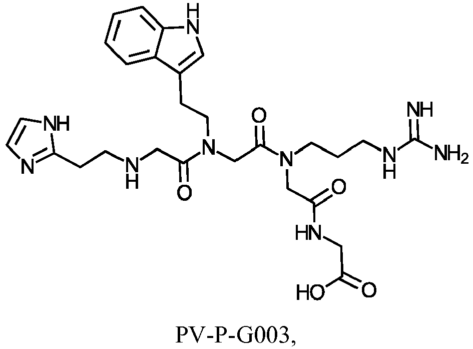

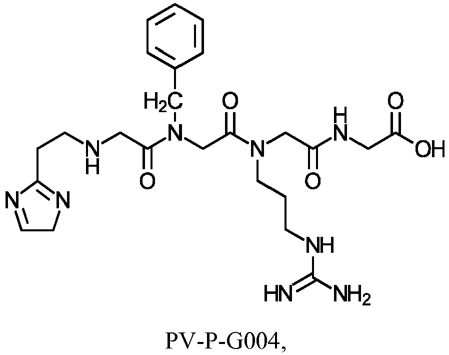

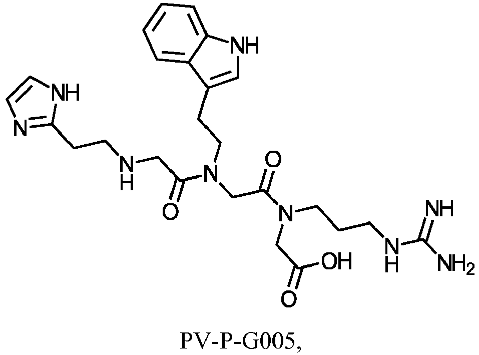

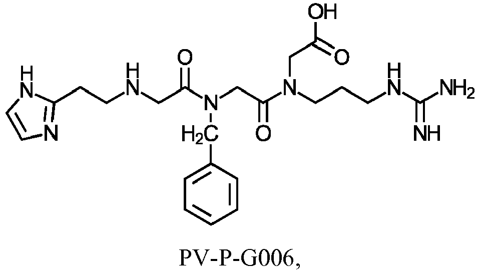

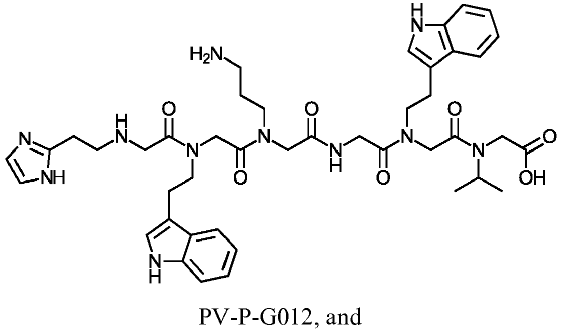

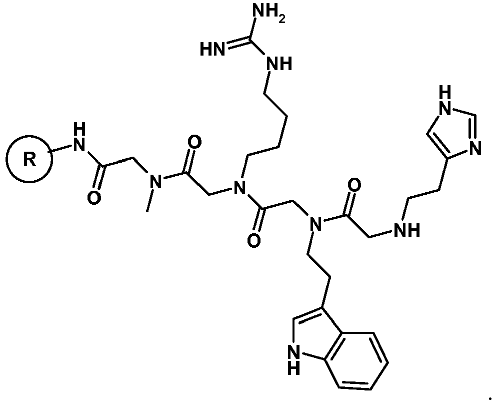

## Patentansprüche

1. Eine Verbindung, die ausgewählt ist aus der Gruppe bestehend aus Verbindungen der Formeln I-IV: 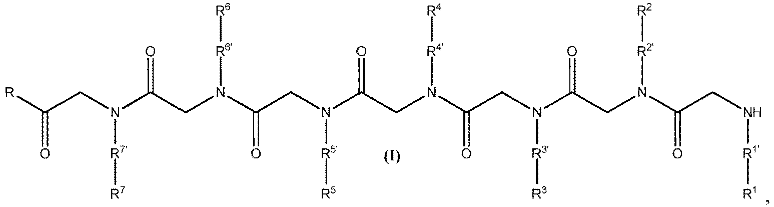 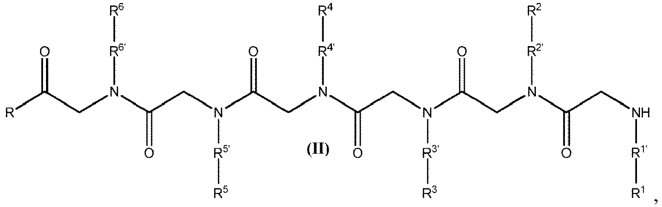 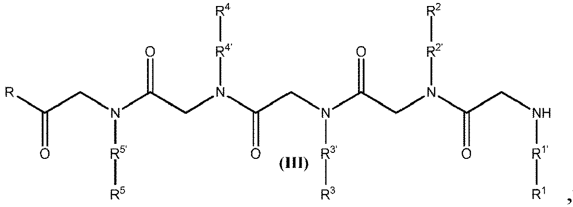 und 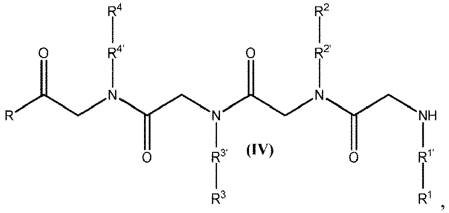 wobei:
R -OH, -NH₂, -OR' oder -NHR' ist, wobei R' ein fester Träger ist und die Verbindung optional über eine Kopplungs-Gruppe an den festen Träger gebunden ist;
R^{1'}, R^{2'}, R^{3'}, R^{5'} und R^{7'} jeweils unabhängig eine C1- bis C4-Alkylengruppe sind;
R^{4'} und R^{6'} jeweils unabhängig fehlen oder eine C1- bis C4-Alkylengruppe sind;
R¹ eine basische Gruppe ist;
R² eine aromatische Gruppe ist;
R³ eine basische Gruppe oder eine hydrophile Gruppe ist;
R⁴ H oder auch, für Verbindungen von Formel IV, eine aliphatische Gruppe, eine aromatische Gruppe, eine acide Gruppe, eine hydrophile Gruppe, eine basische Gruppe oder eine Kopplungs-Gruppe ist;
R⁵ eine aromatische Gruppe, eine basische Gruppe oder auch, für Verbindungen von Formel III, eine aliphatische Gruppe, eine acide Gruppe, eine hydrophile Gruppe oder eine Kopplungs-Gruppe ist;
R⁶ H, eine acide Gruppe oder auch, für Verbindungen von Formel II, eine aliphatische Gruppe, eine aromatische Gruppe, eine hydrophile Gruppe, eine basische Gruppe oder eine Kopplungs-Gruppe ist;
R⁷ H, eine aliphatische Gruppe, eine aromatische Gruppe, eine acide Gruppe, eine hydrophile Gruppe, eine basische Gruppe oder eine Kopplungs-Gruppe ist.

2. Eine Verbindung von Anspruch 1 mit der Struktur von Formel I: 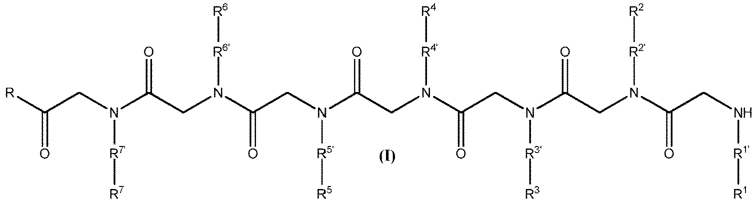 wobei:
R -OH, -NH₂, -OR' oder -NHR' ist, wobei R' ein fester Träger ist und die Verbindung optional über eine Kopplungs-Gruppe an den festen Träger gebunden ist;
R^{1'}, R^{2'}, R^{3'}, R^{5'} und R^{7'} jeweils unabhängig eine C1- bis C4-Alkylengruppe sind;
R^{4'} und R^{6'} jeweils unabhängig fehlen oder eine C1- bis C4-Alkylengruppe sind;
R¹ eine basische Gruppe ist;
R² eine aromatische Gruppe ist;
R³ eine basische Gruppe oder eine hydrophile Gruppe ist;
R⁴ H ist;
R⁵ eine aromatische Gruppe oder eine basische Gruppe ist;
R⁶ H oder eine acide Gruppe ist;
R⁷ H, eine aliphatische Gruppe, eine aromatische Gruppe, eine acide Gruppe, eine hydrophile Gruppe, eine basische Gruppe oder eine Kopplungs-Gruppe ist.

3. Eine Verbindung von Anspruch 1 mit der Struktur von Formel II: 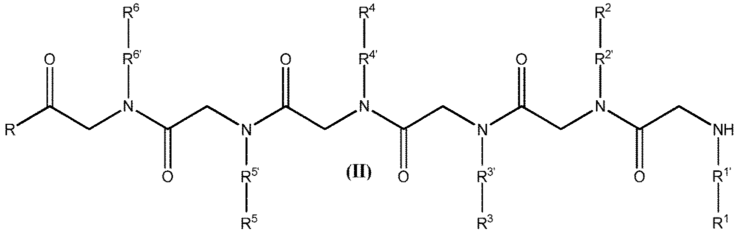 wobei:
R -OH, -NH₂, -OR' oder -NHR' ist, wobei R' ein fester Träger ist und die Verbindung optional über eine Kopplungs-Gruppe an den festen Träger gebunden ist;
R^{1'}, R^{2'}, R^{3'}, R^{5'} und R^{7'} jeweils unabhängig eine C1- bis C4-Alkylengruppe sind;
R^{4'} und R^{6'} jeweils unabhängig fehlen oder eine C1- bis C4-Alkylengruppe sind;
R¹ eine basische Gruppe ist;
R² eine aromatische Gruppe ist;
R³ eine basische Gruppe oder eine hydrophile Gruppe ist;
R⁴ H ist;
R⁵ eine aromatische Gruppe oder eine basische Gruppe ist;
R⁶ H eine acide Gruppe, eine aliphatische Gruppe, eine aromatische Gruppe, eine hydrophile Gruppe, eine basische Gruppe oder eine Kopplungs-Gruppe ist.

4. Eine Verbindung von Anspruch 1 mit der Struktur von Formel III: 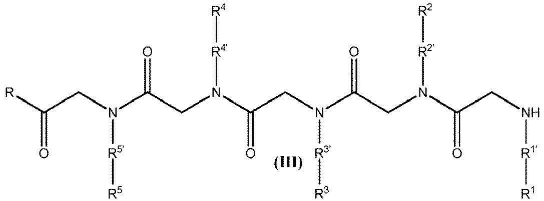 wobei:
R -OH, -NH₂, -OR' oder -NHR' ist, wobei R' ein fester Träger ist und die Verbindung optional über eine Kopplungs-Gruppe an den festen Träger gebunden ist;
R^{1'}, R^{2'}, R^{3'}, R^{5'} und R^{7'} jeweils unabhängig eine C1- bis C4-Alkylengruppe sind;
R^{4'} und R^{6'} jeweils unabhängig fehlen oder eine C1- bis C4-Alkylengruppe sind;
R¹ eine basische Gruppe ist;
R² eine aromatische Gruppe ist;
R³ eine basische Gruppe oder eine hydrophile Gruppe ist;
R⁴ H ist;
R⁵ eine aromatische Gruppe, eine basische Gruppe, eine aliphatische Gruppe, eine acide Gruppe, eine hydrophile Gruppe oder eine Kopplungs-Gruppe ist.

5. Eine Verbindung von Anspruch 1 mit der Struktur von Formel IV: 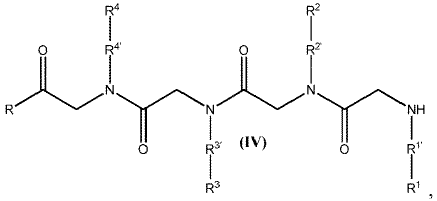 wobei:
R -OH, -NH₂, -OR' oder -NHR' ist, wobei R' ein fester Träger ist und die Verbindung optional über eine Kopplungs-Gruppe an den festen Träger gebunden ist;
R^{1'}, R^{2'}, R^{3'}, R^{5'} und R^{7'} jeweils unabhängig eine C1- bis C4-Alkylengruppe sind;
R^{4'} und R⁶ jeweils unabhängig fehlen oder eine C1- bis C4-Alkylengruppe sind;
R¹ eine basische Gruppe ist;
R² eine aromatische Gruppe ist;
R³ eine basische Gruppe oder eine hydrophile Gruppe ist;
R⁴ H, eine aliphatische Gruppe, eine aromatische Gruppe, eine acide Gruppe, eine hydrophile Gruppe, eine basische Gruppe oder eine Kopplungs-Gruppe ist.

6. Die Verbindung von Anspruch 1, wobei:
R¹ eine basische aromatische Gruppe ist; und/oder
R⁵ eine basische aromatische Gruppe ist.

7. Die Verbindung von Anspruch 1, wobei:
R¹ ausgewählt ist aus der Gruppe bestehend aus Pyridyl-, Chinolyl-, Isochinolyl-, Acridyl-, Pyrazyl-, Chinoxalyl-, Imidazolyl-, Benzimidazolyl-, Puryl-, Pyrazolyl-, Indazolyl-, Pyrimidyl-, Chinazolyl-, Pyridazyl- und Cinnolyl-; und/oder
R² ausgewählt ist aus der Gruppe bestehend aus Phenyl-, Benzyl-, Naphtalyl-, Indolyl- und substituierten Derivaten davon; und/oder
R³ ausgewählt ist aus der Gruppe bestehend aus primärem, sekundärem, tertiärem und quartärem Amin, Guanidyl-, Carbamoyl-, Hydroxyl- und Harnstoff; und/oder R⁴ H ist, und/oder
R⁵ eine aromatische Gruppe, eine basische Gruppe oder eine basische aromatische Gruppe ist.

8. Die Verbindung von Anspruch 1 bis 7, wobei jener feste Träger ein Partikel umfasst.

9. Die Verbindung von Anspruch 1 bis 8, wobei jener feste Träger ein anorganisches Material umfasst oder wobei jener feste Träger ein organisches Polymer-Material umfasst.

10. Ein Verfahren zur Bindung eines Antikörpers oder Antikörper-Fc-Fragments aus einer flüssigen Zusammensetzung, die selbigen/selbiges enthält, umfassend die folgenden Schritte:
(a) Bereitstellen eines festen Trägers, der eine Verbindung von Anspruch 1 bis 9 umfasst, wobei R -OR' oder -NHR' ist und die Verbindung optional über eine Kopplungs-Gruppe an jenen festen Träger gebunden ist;
(b) Kontaktieren jener Zusammensetzung mit jenem festen Träger, sodass jener Antikörper oder jene Fc-Fragmente an jene Verbindung binden; und
(c) Trennen jener flüssigen Zusammensetzung von jenem festen Träger, wobei jener Antikörper oder jenes Fc-Fragment an jenen festen Träger gebunden sind.

11. Das Verfahren von Anspruch 10, wobei jene flüssige Zusammensetzung außerdem mindestens ein proteolytisches Enzym umfasst und/oder wobei jene flüssige Zusammensetzung außerdem eine biologische Flüssigkeit umfasst, wobei jene biologische Flüssigkeit optional Blut, Blutseren, Blutplasma, Gewebe- oder ZellkulturMedien, ein Zelllysat, einen Pflanzenextrakt oder eine Flüssigkeit, die von einem rekombinanten Tier produziert und/oder sekretiert wird, umfasst.

12. Das Verfahren eines der Ansprüche 10 bis 11, wobei jene Kontaktier- und Trenn-Schritte kontinuierlich ausgeführt werden.

13. Das Verfahren von einem der Ansprüche 10 bis 12, außerdem umfassend den folgenden Schritt:
(d) Trennen jenes Antikörpers oder Antikörper-Fc-Fragments von jenem festen Träger.

14. Das Verfahren von einem der Ansprüche 10 bis 13, wobei der Antikörper oder das Antikörper-Fc-Fragment ein IgG-, IgA-, IgE-, IgD-, IgM- oder IgY-Antikörper oder -Antikörperfragment ist, wobei der Antikörper oder das Antikörper-Fc-Fragment optional ein Säuger-Antikörper oder -Antikörper-Fragment ist.

15. Die Verbindung von Anspruch 1-9 oder das Verfahren von Anspruch 10 bis 14, wobei jene Verbindung ausgewählt ist aus der Gruppe bestehend aus:

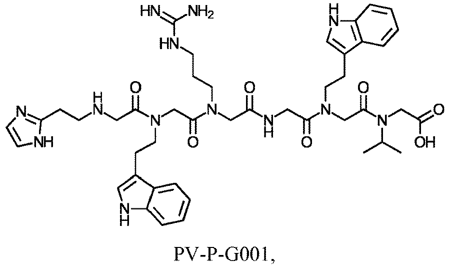

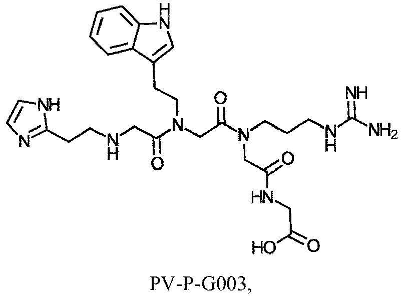

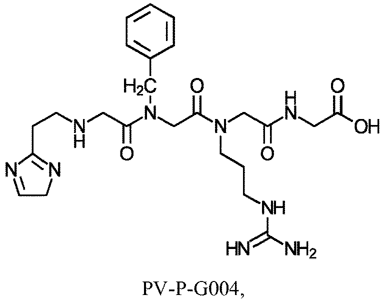

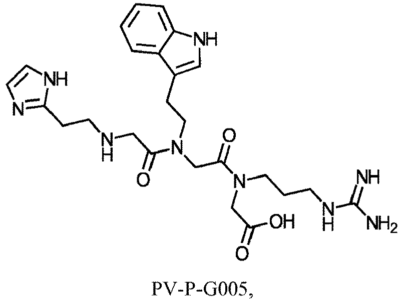

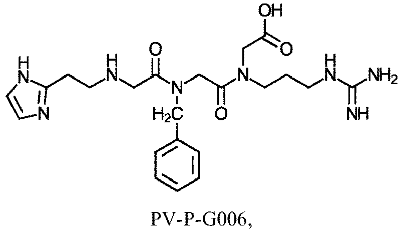

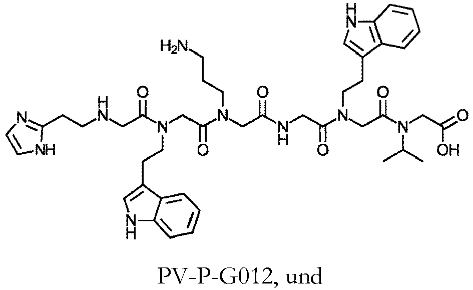

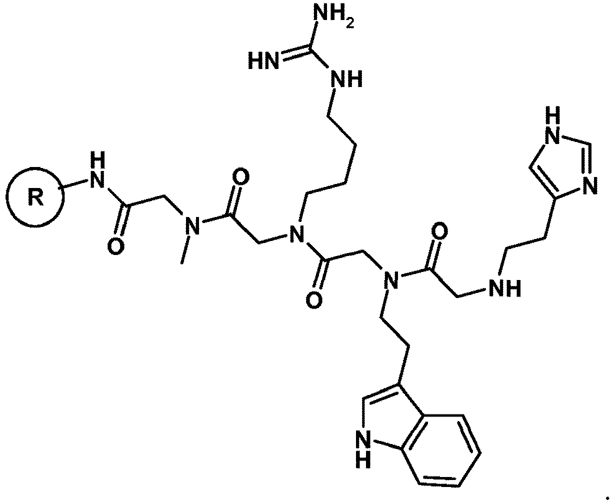

## Revendications

1. - Composé choisi dans le groupe consistant en les composés de **Formules I-IV :** 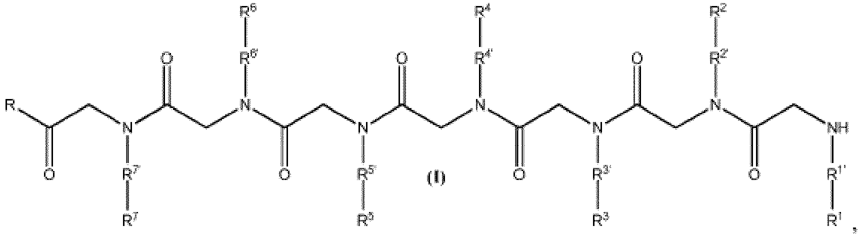 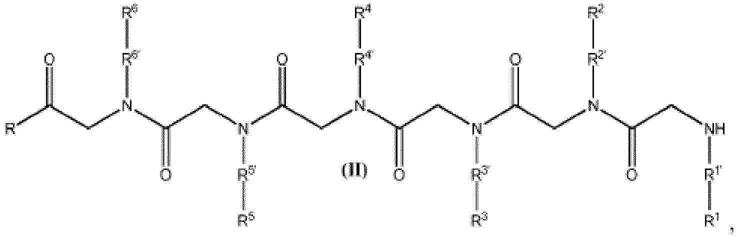 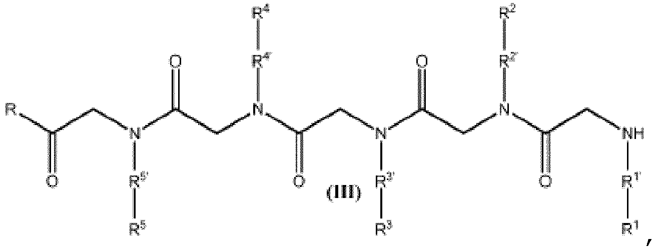 et 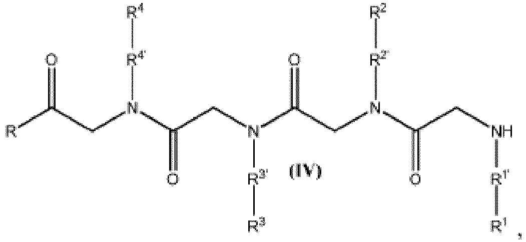 où :
R représente -OH, -NH₂, -OR' ou -NHR', où R' représente un support solide et le composé est facultativement attaché au support solide par l'intermédiaire d'un groupe de couplage ;
R^{1'}, R^{2'}, R^{3'}, R^{5'} et R^{7'} représentent chacun indépendamment un groupe alkylène en C1 à C4 ;
R^{4'} et R^{6'} sont chacun indépendamment absents ou représentent un groupe alkylène en C1 à C4 ;
R¹ représente un groupe basique ;
R² représente un groupe aromatique ;
R³ représente un groupe basique ou un groupe hydrophile ;
R⁴ représente H ou également pour les composés de **Formule IV,** un groupe aliphatique, un groupe aromatique, un groupe acide, un groupe hydrophile, un groupe basique ou un groupe de couplage ;
R⁵ représente un groupe aromatique, un groupe basique ou également pour les composés de **Formule III,** un groupe aliphatique, un groupe acide, un groupe hydrophile ou un groupe de couplage ;
R⁶ représente H, un groupe acide, ou également pour les composés de **Formule II,** un groupe aliphatique, un groupe aromatique, un groupe hydrophile, un groupe basique ou un groupe de couplage;
R⁷ représente H, un groupe aliphatique, un groupe aromatique, un groupe acide, un groupe hydrophile, un groupe basique ou un groupe de couplage.

2. - Composé selon la revendication 1 ayant une structure de **Formule I :** 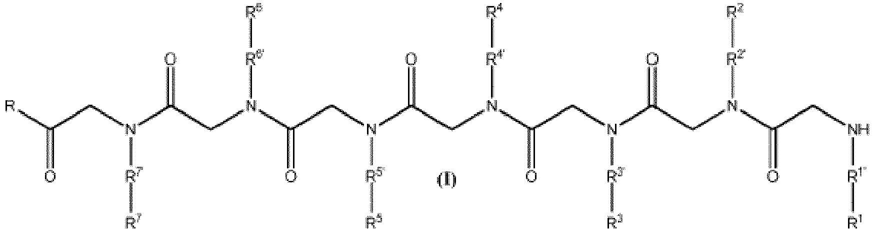 où :
R représente -OH, -NH₂, -OR' ou -NHR', où R' représente un support solide et le composé est facultativement attaché au support solide par l'intermédiaire d'un groupe de couplage ;
R^{1'}, R^{2'}, R^{3'}, R^{5'} et R^{7'} représentent chacun indépendamment un groupe alkylène en C1 à C4 ;
R^{4'} et R^{6'} sont chacun indépendamment absents ou représentent un groupe alkylène en C1 à C4 ;
R¹ représente un groupe basique ;
R² représente un groupe aromatique ;
R³ représente un groupe basique ou un groupe hydrophile ;
R⁴ représente H ;
R⁵ représente un groupe aromatique ou un groupe basique ;
R⁶ représente H ou un groupe acide ;
R⁷ représente H, un groupe aliphatique, un groupe aromatique, un groupe acide, un groupe hydrophile, un groupe basique ou un groupe de couplage.

3. - Composé selon la revendication 1 ayant la structure de **Formule II** : 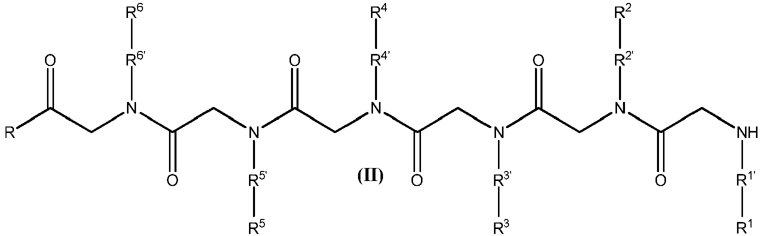 où :
R représente -OH, -NH₂, -OR' ou -NHR', où R' représente un support solide et le composé est facultativement attaché au support solide par l'intermédiaire d'un groupe de couplage ;
R^{1'}, R^{2'}, R^{3'}, R^{5'} et R^{7'} représentent chacun indépendamment un groupe alkylène en C1 à C4 ;
R^{4'} et R^{6'} sont chacun indépendamment absents ou représentent un groupe alkylène en C1 à C4 ;
R¹ représente un groupe basique ;
R² représente un groupe aromatique ;
R³ représente un groupe basique ou un groupe hydrophile ;
R⁴ représente H ;
R⁵ représente un groupe aromatique ou un groupe basique ;
R⁶ représente H, un groupe acide, un groupe aliphatique, un groupe aromatique, un groupe hydrophile, un groupe basique ou un groupe de couplage.

4. - Composé selon la revendication 1 ayant la structure de **Formule III** : 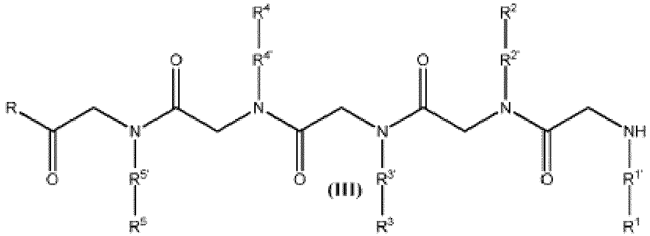 où :
R représente -OH, -NH₂, -OR' ou -NHR', où R' représente un support solide et le composé est facultativement attaché au support solide par l'intermédiaire d'un groupe de couplage ;
R^{1'}, R^{2'}, R^{3'}, R^{5'} et R^{7'} représentent chacun indépendamment un groupe alkylène en C1 à C4 ;
R^{4'} et R^{6'} sont chacun indépendamment absents ou représentent un groupe alkylène en C1 à C4 ;
R¹ représente un groupe basique ;
R² représente un groupe aromatique ;
R³ représente un groupe basique ou un groupe hydrophile ;
R⁴ représente H ;
R⁵ représente un groupe aromatique, un groupe basique, un groupe aliphatique, un groupe acide, un groupe hydrophile ou un groupe de couplage.

5. - Composé selon la revendication 1 ayant la structure de **Formule IV** : 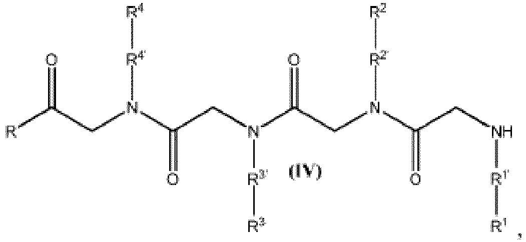 où :
R représente -OH, -NH₂, -OR' ou -NHR', où R' représente un support solide et le composé est facultativement attaché au support solide par l'intermédiaire d'un groupe de couplage ;
R^{1'}, R^{2'}, R^{3'}, R^{5'} et R^{7'} représentent chacun indépendamment un groupe alkylène en C1 à C4 ;
R^{4'} et R^{6'} sont chacun indépendamment absents ou représentent un groupe alkylène en C1 à C4 ;
R¹ représente un groupe basique ;
R² représente un groupe aromatique ;
R³ représente un groupe basique ou un groupe hydrophile ;
R⁴ représente H, un groupe aliphatique, un groupe aromatique, un groupe acide, un groupe hydrophile, un groupe basique ou un groupe de couplage.

6. - Composé selon la revendication 1, dans lequel :
R¹ représente un groupe aromatique basique ; et/ou
R⁵ représente un groupe aromatique basique.

7. - Composé selon la revendication 1, dans lequel :
R¹ est choisi dans le groupe consistant en pyridinyl-, quinolinyl-, isoquinolinyl-, acridinyl-, pyrazinyl-, quinoxalinyl-, imidazolyl-, benzimidazolyl-, purinyl-, pyrazolyl-, indazolyl-, pyrimidinyl-, quinazolinyl-, pyridazinyl- et cinnolinyl- ; et/ou
R² est choisi dans le groupe consistant en phényl-, benzyl-, naphtalyl-, indolyl- et des dérivés substitués de ceux-ci ; et/ou
R³ est choisi dans le groupe consistant en amine primaire, secondaire, tertiaire et quaternaire, guanidinyl-, carbamoyl-, hydroxyl- et urée ; et/ou
R⁴ représente H, et/ou
R⁵ représente un groupe aromatique, un groupe basique ou un groupe aromatique basique.

8. - Composé selon l'une des revendications 1 à 7, dans lequel ledit support solide comprend une particule.

9. - Composé selon l'une des revendications 1 à 8, dans lequel ledit support solide comprend une matière inorganique, ou
dans lequel ledit support solide comprend une matière polymère organique.

10. - Procédé de liaison d'un anticorps ou d'un fragment Fc d'anticorps provenant d'une composition liquide le contenant, comprenant les étapes de :
(a) se procurer un support solide comprenant un composé selon l'une des revendications 1 à 9, où R représente -OR' ou -NHR' et le composé est facultativement attaché audit support solide par l'intermédiaire d'un groupe de couplage ;
(b) mettre en contact ladite composition avec ledit support solide de telle sorte que ledit anticorps ou fragments Fc se lient audit composé ; et
(c) séparer ladite composition liquide dudit support solide, avec ledit anticorps ou fragment Fc lié audit support solide.

11. - Procédé selon la revendication 10, dans lequel ladite composition liquide comprend de plus au moins une enzyme protéolytique et/ou ladite composition liquide comprend de plus un fluide biologique facultativement dans lequel ledit fluide biologique comprend le sang, les sérums sanguins, le plasma sanguin, un milieu de culture tissulaire ou cellulaire, un lysat cellulaire, un extrait de plante ou un fluide produit et/ou sécrété par un animal recombinant.

12. - Procédé selon l'une quelconque des revendications 10 à 11, dans lequel lesdites étapes de mise en contact et de séparation sont effectuées en continu.

13. - Procédé selon l'une quelconque des revendications 10 à 12, comprenant de plus l'étape de :
(d) séparer ledit anticorps ou fragment Fc d'anticorps à partir dudit support solide.

14. - Procédé selon l'une quelconque des revendications 10 à 13, dans lequel l'anticorps ou fragment Fc d'anticorps est un anticorps ou fragment d'anticorps IgG, IgA, IgE, IgD, IgM ou IgY, facultativement dans lequel l'anticorps ou fragment Fc d'anticorps est un anticorps ou fragment d'anticorps de mammifère.

15. - Composé selon l'une des revendications 1 à 9 ou procédé selon l'une des revendications 10 à 14, dans lequel ledit composé est choisi dans le groupe consistant en :
